# EUROPEAN PATENT SPECIFICATION

(11) **EP 4 051 195 B1**
(45) Date of publication and mention of the grant of the patent: **26.07.2023**
(21) Application number: 21822324.6
(22) Date of filing: 10.06.2021
(51) Int. Cl.: A61F 9/08, A61N 7/00, A61N 1/36, A61N 5/02

(54) **UNIT FOR NON-INVASIVE STIMULATION OF THE VISUAL CORTEX CELLS IN SEVERE VISUAL IMPAIRMENT**
EINHEIT ZUR NICHT-INVASIVEN STIMULATION DER SEHRINDENZELLEN BEI STARKER SEHBEHINDERUNG
UNITÉ DE STIMULATION NON EFFRACTIVE DES CELLULES DU CORTEX VISUEL POUR DÉFICIENCE VISUELLE SÉVÈRE

(30) Priority: 10.06.2020 CZ 20200337
(43) Date of publication of application: 07.09.2022
(73) Proprietor: Ceské vysoké ucení technické v Praze, 16000 Praha 6- Dejvice (CZ)
(72) Inventor: HANA, Karel, 10100 Praha 10, Vrsovice (CZ); CHOD, Jiri, 15800 Praha 5, Stodulky (CZ); LESTAK, Jan, 27351 Horni Bezdekov (CZ); ROSINA, Jozef, 16200 Praha 6, Brevnov (CZ)
(74) Representative: Kratochvil, Vaclav
(86) International application number: PCT/IB2021/055086
(87) International publication number: WO 2021/250596

(56) References cited:
- JP-A- 2008 093 286
- US-A1- 2003 145 864
- US-A1- 2014 222 103
- Piedade M., Gerald J., Sousa L.A., Tavares G., Tomas P.: "Visual neuroprosthesis: a non invasive system for stimulating the cortex", IEEE TRANSACTIONS ON CIRCUITS AND SYSTEMS PART I: REGULAR PAPERS., IEEE SERVICE CENTER, NEW YORK, NY., US, vol. 52, no. 12, 1 December 2005 (2005-12-01), pages 2648-2662, XP055886580, US ISSN: 1057-7122, DOI: 10.1109/TCSI.2005.857923

## Description

### Technical Field

The technical solution refers to a unit for non-invasive stimulation of visual cortex cells in severe visual impairment, employing telecommunication technology with electronics and sensors, computer technology and wireless communication; specifically, the solution relates to a unit for non-invasive transcranial stimulation using a specifically modified electronic module and the communication thereof with other devices. Therefore, this is a creation of a "bionic eye" for a person concerned.

### Background Art

The effort to create a "bionic" eye using electronics to substitute sight is a logical outcome of state of the art. It will still represent the only effective way to substitute sight until the genetic manipulation era. The conditions are similar to the at present mastered conditions for hearing compensations; however, these are infinitely more complex regarding the significant specifics of vision, perception, and processing thereof by a human brain.

In principle, this is an electronic image sensor, i.e., a chip, a complete camera, individual sensitive sensors, etc., signal of which is processed by a "video chip" - an analogy of image processing for transmission or display to signals that the brain is capable of receiving. These signals usually represent electrical current modulated in different ways to stimulate the appropriate response. The connection is solved in different ways, either by connecting to the original pathway at the level of the retina, optic nerve or the electrodes are introduced directly into the brain as fine electrodes of various designs. Thus, known embodiments always solve the interconnection using electrodes at the level of subcortical or cortical centres in the brain.

Currently, four bionic eye systems have received marketing approval in Europe and the US, wherein a number of others have undergone preclinical and clinical trials, reflecting the established safety profile for permanent stimulation. This progress demonstrates an effort to help blind patients, hoping for tangible and measurable help.

The microchip with electrodes itself may be implanted intraocularly, i.e., it provides stimulation epiretinally, subretinally, or suprachorioideally.

This represents the most significant complication from the patient's point of view and requires extensive invasive interventions into the eye itself, nerve connections thereof to the brain or directly into the brain, with all possible consequences. Document US-A-2003/0145864 discloses the most relevant prior art.

### Summary of Invention The invention is defined in claim 1.

The shortcomings mentioned above are eliminated mainly by the unit for transcranial - non-invasive stimulation of visual cortex cells in severe visual impairment, employing telecommunication technology with electronics and sensors, computer technology, and wireless communication. The system of this disclosure consists in that it comprises an image sensor connected to a video chip to convert the image signal to signals capable of stimulating the brain at a frequency of 5 to 100 GHz, and, further, comprises a supporting structure provided with a transmitting sensor, another transmitting sensor for position stabilisation, a position stabilisation unit maintaining the data flow direction to the actual brain cells, maintaining the position stabilisation in cooperation with a sensing sensor for feedback sensor, all of which are controlled by a processor so that the information transmission is always optimal.

The system is complemented by a communication module capable of communicating with a remote monitoring centre using mobile LAN and WAN networks, especially 2G to 5G networks, and, at the same time, providing the possibility of software modifications to the system. Power is included with the unit and is capable of continuous, wireless recharging. If necessary, image data processing can be taken over at the remote centre and sent back to the unit. What is important is the direct transmission of the image information to the brain without internal electrodes implanted in the brain using appropriate frequencies and suitable modulation, resulting in stimulating neurons.

The summary of the disclosure is precise non-invasive - transcranial stimulation of neurons of the visual brain cortex. The system uses a combination of signals from the electromagnetic spectrum at frequencies from 5 to 100 GHz, to directly stimulate parts of the brain. The support unit also includes a set of sound and ultrasound wave generators and sensors to help focus the main bundle and support stimulation of the neurons themselves. The stimulation itself is spot focused by an antenna assembly of the transmitting unit. This is placed outside the skull; however, it focuses the section in the brain spottily. The sensing image sensor itself - the image sensor, i.e., the camera - is detachable and can be fitted, for example, into glasses, or it can be part of an extension of the supporting structure, e.g., fitted to a wearer's head, to the position needed to acquire the relevant data.

The exact localisation of the selected neurons can be determined, e.g., by functional magnetic resonance on a visual stimulus as an input calibration or directly during tests of the unit itself.

Visual impairment and blindness remain significant public health problems worldwide. The World Health Organization (WHO) estimates that there are approximately 253 million people with visual impairment worldwide: 36 million were blind, and 217 million had moderate to severe visual impairment in 2015. Given this information, the usage of the proposed device could be huge.

### Brief Description of Drawings

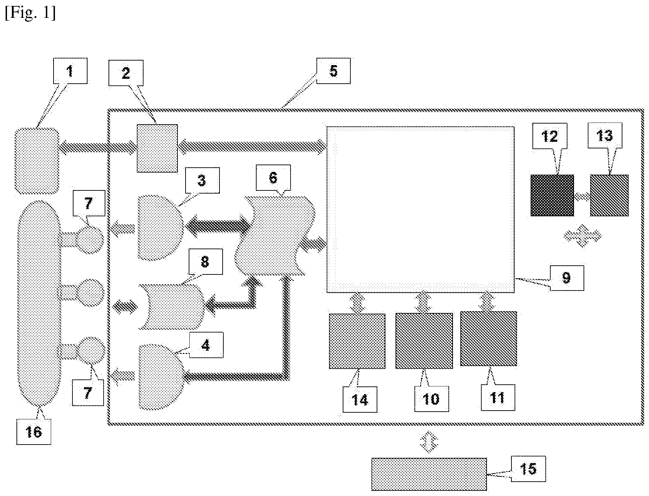

The technical solution is explained in more detail in the attached drawing, wherein [Fig.1] shows an overview diagram of the solution and communication and shows a block diagram of the sensor network, unit modules, and communication thereof.

### Description of Embodiments

The example of the unit arrangement for non-invasive stimulation of visual cortex cells according to the present disclosure is shown as the block diagram in the accompanying drawing [Fig.1]. The unit of the image sensor 1 is wired/wirelessly connected to the video chip 2 and then connected to the control processor 9, to which the first transmitting sensor 3 of the antenna assembly and the second transmitting sensor 4 of the position stabilisation are connected via the position stabilisation unit 6, in connection with the receiving sensor 8 of the position stabilisation feedback, connected to a structure of the carrier 16 for the active/passive positioning sensors 7 fixed/implanted on the head of a subject and carrying the active/passive sensors 7 of the position stabilisation feedback. The assembly is completed with the LAN communication module 10 and the WAN communication module 11 along with the GNSS unit 14, all connected to the control processor 9. All of these are powered by a battery, i.e., the power supply unit 12 charged by the rechargeable module 13. All of these are enclosed within the mechanical supporting structure 5, wherein the assembly can also communicate with the mobile unit 15.

### Industrial Applicability

The unit for non-invasive stimulation of visual cortex cells in severe visual impairment will find the application thereof for visually impaired people, both blind and moderate to severe visual impairment.

### Reference Signs List

1 Image sensor (camera)
2 Video chip
3 Transmitting sensor A (antennae), self-stimulation
4 Transmitting sensor B, position stabilisation
5 Unit supporting structure
6 Position stabilisation unit (gyroscopic and/or floating)
7 Position stabilisation feedback sensors - passive/active (transmitting)
8 Position stabilisation feedback sensors - receiving
9 Processor
10 LAN communication module
11 WAN communication module
12 Power unit (battery)
13 Rechargeable battery module
14 GNSS unit (Galileo, GLONASS, GPS...)
15 Mobile unit (phone, tablet...) to control and set the function
16 Active/passive positioning sensor carrier fixed/implanted on the subject's head

## Claims

1. A unit for non-invasive stimulation of visual cortex cells in severe visual impairment comprising an image sensor and means for stimulating visual cortex cells, wherein the means for stimulating comprises a control processor (9), and wherein the image sensor (1) comprises a video chip (2) connected to the control processor (9) to convert an image signal to signals capable of stimulating a brain at a frequency of 5 to 100 GHz, to which a first transmitting sensor (3) for the stimulation, a second transmitting sensor (4) of the position stabilisation and a receiving sensor (8) of a the- position stabilisation feedback are connected via a position stabilisation unit (6), wherein said sensors are connected to a carrier (16) of active/passive sensors (7) via transmitting active/passive sensors (7) of the position stabilisation feedback, wherein a communication module is connected to the control processor (9).

2. The unit according to claim 1, ***characterised in that*** the communication module is selected from the group consisting of a LAN communication module (10), a WAN communication module (11), and a GNSS unit (14), which are interconnected to a mobile unit (15).

3. The unit according to claim 1 or 2, ***characterised in that*** a power supply unit (12) with a rechargeable module (13) is connected to the control processor (9).

4. The unit according to any one of claims 2 to 3, ***characterised in that*** the video chip (2), the control processor (9), the position stabilisation unit (6), the first transmitting sensor (3), the second transmitting sensor (4), the receiving sensor (8), the LAN communication module (10), the WAN communication module (11), the GNSS unit (14), the power supply unit (12), and the rechargeable module (13) are placed within the supporting structure (5). 1

## Patentansprüche

1. Eine Einheit zur nicht-invasiven Stimulation von Zellen der Sehrinde des Gehirns bei schweren Sehstörungen, umfassend einen Bildsensor und Mittel zur Stimulation von Zellen der Sehrinde des Gehirns, wobei der Bildsensor (1) einen Videochip (2) enthält, verbunden mit einem Steuerprozessor (9) zur Umwandlung des Bildsignals in Signale, die das Gehirn mit einer Frequenz von 5 bis 100 GHz stimulieren können, an den über die Einheit (6) der Lagestabilisierung ein erster Sendesensor (3) eigener Stimulation, ein zweiter Sendesensor (4) der Lagestabilisierung und ein Empfangssensor (8) der Rückmeldung der Lagestabilisierung angeschlossen sind, die über sendende aktive/passive Sensoren (7) der Rückmeldung der Lagestabilisierung mit einem Träger (16) der aktiven/passiven Sensoren (7) verbunden sind, wobei ein Kommunikationsmodul mit dem Steuerprozessor (9) verbunden ist.

2. Einheit nach Anspruch 1, ***gekennzeichnet dadurch, dass*** das Kommunikationsmodul aus der Gruppe LAN-Kommunikationsmodul (10), WAN-Kommunikationsmodul (11) und GNSS-Einheit (14) ausgewählt ist, die mit einer mobilen Einheit (15) verbunden sind.

3. Einheit nach Anspruch 1 oder 2, ***gekennzeichnet dadurch, dass*** an den Steuerprozessor (9) eine Einspeiseeinheit (12) mit einem Lademodul (13) angeschlossen ist.

4. Einheit nach einem der Ansprüche 2 bis 3, ***gekennzeichnet dadurch, dass*** der Videochip (2), der Steuerprozessor (9), die Einheit (6) der Lagestabilisierung, der erste Sendesensor (3), der zweite Sendesensor (4), der Empfangssensor (8), das LAN-Kommunikationsmodul (10), das WAN-Kommunikationsmodul (11), die GNSS-Einheit (14), die Einspeiseeinheit (12) und das Lademodul (13) in einer Tragkonstruktion (5) angeordnet sind.

## Revendications

1. Unité pour une stimulation non invasive de cellules de l'écorce visuelle de cerveau dans les troubles visuels sévères contenant un capteur d'image et des moyens pour stimuler les cellules de l'écorce visuelle de cerveau, le capteur (1) d'image contenant une puce (2) vidéo reliée à un processeur (9) de commande pour convertir le signal d'image en signaux aptes à stimuler le cerveau d'une fréquence de 5 à 100 GHz, auquel un premier capteur (3) d'émission de la stimulation elle-même, un deuxième capteur (4) d'émission de stabilisation de position et un capteur (8) de réception de retour de stabilisation de position sont reliés via une unité (6) de stabilisation de position et qui sont reliés à un support (16) de capteurs (7) actifs/passifs via des capteurs (7) actifs/passifs de réponse de stabilisation de position, un module de communication étant relié au processeur (9) de commande.

2. Unité selon la revendication 1 ***caractérisée en ce que*** le module de communication est choisi dans le groupe comprenant un module (10) de communication LAN, un module (11) de communication WAN et une unité (14) GNSS qui sont reliés à une unité (15) mobile.

3. Unité selon la revendication 1 ou 2 ***caractérisée en ce qu'*** une unité (12) d'alimentation avec un module (13) de recharge est reliée au processeur (9) de commande.

4. Unité selon l'une quelconque des revendications 2 à 3 ***caractérisée en ce que*** la puce vidéo (2), le processeur (9) de commande, l'unité (6) de stabilisation de position ; le premier capteur (3) d'émission, deuxième capteur (4) d'émission, un capteur (8) de réception, le module (10) de communication LAN, le module (11) de communication WAN, l'unité (14) GNSS, l'unité (12) d'alimentation et le module (13) de recharge sont placés dans une structure (5) porteuse.
